# EUROPEAN PATENT APPLICATION

(11) **EP 4 740 832 A1**
(43) Date of publication of application: **13.05.2026**
(21) Application number: 24211036.9
(22) Date of filing: 06.11.2024
(51) Int. Cl.: A61B 1/313

(54) **ELECTRONICS MODULE FOR INTEGRATION INTO A HANDGRIP OF A MEDICAL INSTRUMENT, HANDGRIP AND MEDICAL INSTRUMENT**

(71) Applicant: Olympus Surgical Technologies Europe, 22045 Hamburg (DE)
(72) Inventor: JUNGBAUER, Sebastian, 22045 Hamburg (DE); MÜCKNER, Andreas, 22045 Hamburg (DE)
(74) Representative: Weickmann & Weickmann PartmbB

(57) **Abstract**

The present invention relates to an electronics module (20), in particular sensor data logger, for integration into a handgrip (10) of a medical instrument, comprising a two-part printed circuit board (24), PCB, wherein a first part (24a) of the PCB (24) is at least as wide as a second part (24b) of the PCB (24), the two parts (24a, 24b) are arranged in such a manner that the second part (24b) is positioned in a plane different from the plane of the first part (24a), in a top view onto the first part (24a), the second part (24b) at least concerning a width direction (W) is contained within the width of the first part (24a), and the two parts (14a, 24b) are interconnected, electronic components (22) provided to the PCB (24); and an encapsulation material (26) encapsulating the PCB (24), wherein the encapsulation material (26) is resilient to sterilization processes. The invention also relates to a handgrip (10) for a medical instrument comprising such an electronics module (20) and a medical instrument comprising such a handgrip.

## Description

The present invention relates to an electronics module, in particular a sensor data logger, for integration into a handgrip of a medical instrument, a handgrip of a medical instrument comprising such an electronics module, and a medical instrument, comprising such a handgrip.

Recently, electronics modules have been developed, which are to be integrated into handgrips of medical instruments, such as for example sensor data logger devices, which are adapted to record usage data of the corresponding medical devices and to store and/or transmit said data for further processing. One particular example for such a device concerns the integration of accelerometers into respective handgrips, which can detect drops and impacts on medical instruments, in order to monitor their usage and predict possible damages thereof. However, other types of sensors may also be employed to ensure that the corresponding device has not been subjected to potentially harmful conditions.

Electronics modules of these types are preferably capable of standalone operation, such that they can perform their functionality even if the handgrip itself is not mechanically and electrically connected to a superordinate structure of the medical instrument, which requires the integration of an independent power source. It also has to be kept in mind that over their lifetime, such handgrips regularly have to be subjected to sterilization processes, such as steam sterilization, plasma sterilization or treatment in an automated washer disinfector, which might damage electronic components if they are not protected in a suitable manner.

There is therefore the need for electronics modules for integration into handgrips of medical instruments, which on the one hand fulfil the requirements on size and geometry to be able to be integrated into the small inner spaces of such handgrips, while on the other hand it is necessary that the electronics modules will not be impaired or damaged during sterilization processes of the handgrips themselves.

It is therefore the object of the present invention to provide such an electronics module which has geometrical dimensions that make it suitable for integration into the handgrip of a medical instrument where it can perform its intended functionality and at the same time is resistant to frequent sterilization processes which are performed on the handgrip over its lifespan.

For this purpose, the present invention provides an electronics module, in particular a sensor data logger, for integration into a handgrip of a medical instrument, comprising a two-part printed circuit board, PCB, wherein a first part of the PCB is at least as wide as the second part of the PCB, the two parts are arranged in such a manner that the second part is positioned in a plane different from the plane of the first part, in a top view onto the first part the second part at least concerning a width direction is contained within the width of the first part and the two parts are interconnected, wherein the electronics module further comprises electronic components provided to the PCB, and an encapsulation material encapsulating the PCB, wherein the encapsulation material is resilient to sterilization processes.

By thus distributing the PCB with its electronic components onto two different planes, which may for example be stacked on top of one another with respect to the width direction of the first part, and by furthermore encapsulating the entire PCB into a suitable encapsulation material, both a compact design thereof and a high resiliency to external influences and in particular sterilization processes can be achieved.

It shall be pointed out that in certain embodiments, selected electronic components may project out of the encapsulation material, such as for example antennas for a communication with external communication devices, which may extend beyond the PCBs and are as such not as sensitive to sterilization processes as other electronics components.

In order to be able to achieve the above-mentioned standalone operation of the electronics module according to the present invention, said module may further comprise at least one energy storage unit, in particular a battery, for example a primary coin cell, for powering the electronic components. In different embodiments, other types of energy storage units may also be provided, such as for example rechargeable batteries and/or supercapacitors. By providing such energy storage units, no electrical coupling between the PCB of the electronics module and an external power source or the main medical instrument is required.

Independent of the concrete type of energy storage unit employed in the electronics module, said at least one energy storage unit may also be encapsulated by the encapsulation material, such that in the case of rechargeable energy storage units, either a charging interface has to extend out of the encapsulation material or the capability for contactless charging has to be provided.

While different encapsulation materials are known in the art, which are resilient to sterilization processes, such as steam sterilization, plasma sterilization and treatments in automated washer disinfectors, the encapsulation material of the module according to the present invention may for example be a silicone compound or a duromer plastic compound, which are both relatively cheap and easy to apply on the respective components to be protected. Such encapsulation materials are preferably applied in low-temperature processes, such that the electronic components as well as possible solder connections and energy storage units are not deteriorated.

In order to be able to not only achieve a standalone operation of the electronics module, but to also be able to transmit data from the electronic components to an external communication device, the electronic components may comprise an antenna system and a radio function for wireless communication with said external communication unit integrated in the electronic components. For this purpose, known communication standards and frequencies may be used, or a proprietary protocol may be employed, and different transmission frequencies with corresponding dimensions of the antenna system may be chosen, depending on the concrete implementation of the data transfer.

As already briefly mentioned above, the electronic components may in particular be adapted for a regular collection of sensor data by means of at least one sensor unit, which is comprised therein, for example concerning temperature, pressure, humidity, acceleration, bending forces, mechanical forces and/or the presence of chemical substances. By providing such a sensor unit, usage conditions of the corresponding medical instrument may be logged over time in order to ensure that said instrument has not been subjected to irregular conditions, which might cause damage thereto.

Concerning the arrangement of the first and second parts of the PCB, the plane of the first part of the PCB may in particular be parallel to the plane of the second part of the PCB, however, different angles between the two parts are also conceivable, depending on the concrete geometrical constraints given on the electronics module at its mounting position within the corresponding handgrip.

While the first part and the second part of the PCB may furthermore be formed as two components of a single flexible or bent PCB, they may also be embodied by separate boards interconnected at a longitudinal end region thereof, in particular by means of a flexible PCB connection. However, other types of connections between the two parts of the PCB may also be implemented, such as wiring in a longitudinally central part thereof.

According to a further aspect, the present invention relates to a handgrip of a medical instrument, comprising an outer shell part delimiting an inner space of the handgrip, and an electronics module according to the present invention as just discussed positioned in the inner space of the handgrip, preferably positioned next to the inner wall of the outer shell part. Additionally, a partitioning wall inside the inner space of the handgrip may be provided, which together with the outer shell part defines a compartment of the inner space, in which the electronics module is housed. Additional partition walls at the longitudinal ends of the compartment may additionally be provided, in order to further encapsulate the electronics module on all sides thereof, wherein at least one of the partition walls may be opened and closed for improved accessibility to the electronics module.

In particular, the encapsulation material may substantially fill the entire compartment for at least a longitudinal section of the inner space of the handgrip and/or the partitioning wall may be positioned inside the inner space of the handgrip in such a manner that in a cross-section view, the compartment accounts for less than half of the cross-section area of the inner space.

Additionally or alternatively, the outer shell part may have a substantially circular cross-section, such that the electronics module, if positioned at a radially outer position adjacent to the inner wall of said outer shell part, may in particular have an encapsulation in the form of a cylinder cut-out.

Furthermore, the present invention relates to a medical instrument, in particular a video laparoscope, comprising a handgrip according to the present invention as just described.

Further features and advantages of the present invention will become even clearer from the following description of an embodiment thereof, when viewed together with the accompanying drawings. These show in particular:
- Figure 1: a cross-section view of a handgrip of a medical instrument according to the present invention comprising an electronics module in its inner space, and
- Figure 2: a side view of the electronics module of figure 1.

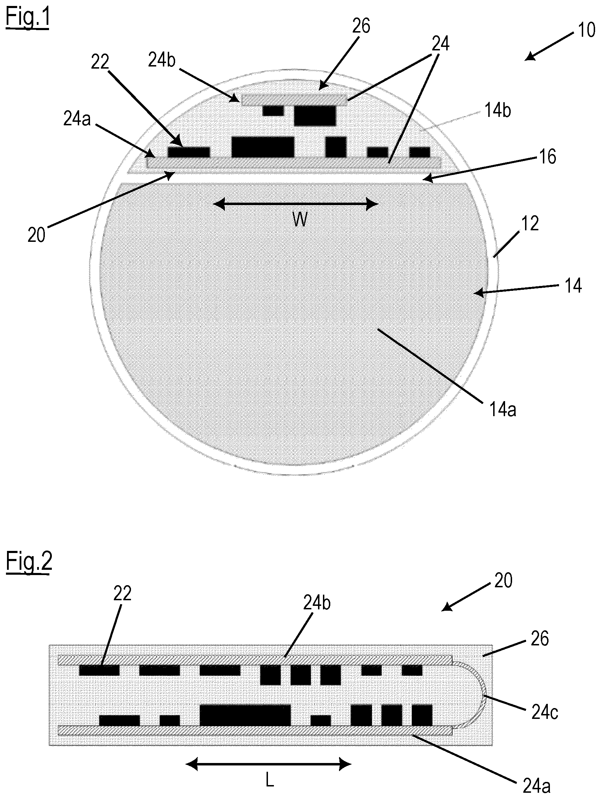

Figure 1 shows a handgrip 10 of a medical instrument which may be gripped by a user, wherein the medical instrument may for example be a video laparoscope, in which typically light guides and further video components as well as mechanical components pass through the handgrip 10.

For this purpose, the handgrip 10 comprises an outer shell 12, which may for example be made of a plastic material and which delimits an inner space 14 of the handgrip 10. Said inner space 14 is divided into a main part 14a and a compartment 14b by means of a partitioning wall 16, wherein in the cross-section view of figure 1 it can be seen that the cross-section area taken up by the compartment 14b is smaller than the remaining free space of the main part 14a, in which for example light guides and video connections as well as mechanical components may be installed. Furthermore, a longitudinal and a width direction of the handgrip 10 are represented in figures 1 and 2 by arrows L and W, respectively.

In the compartment 14b, which may also be further delimited at its longitudinal ends in the extension direction of the handgrip 10, an electronics module according to the present invention is housed, which is generally denoted with reference numeral 20 and again shown in figure 2 in a schematic side view.

Said electronics module 20 may for example be or comprise a sensor data logger, in turn comprising electronic components such as sensors, a control unit, an antenna system and battery cells, which are commonly referred to with reference number 22 and which are provided to a two-part printed circuit board, PCB, 24. In the embodiment shown in figures 1 and 2, said two-part PCB comprises a wide first part 24a and a narrow second part 24b with respect to the width direction W, which extend in parallel planes and which are fitted to the available space in the compartment 14b inside the handgrip 10. Herein, in a top view onto the first part 24a, in Figure 1 corresponding to a radial direction towards the center of the circular outer shell 12, the second part 24b is fully contained within the first part 24a, both concerning the longitudinal direction L and the width direction W.

In one of their longitudinal end regions, the first and second parts 24a, 24b of the PCB 24 are connected by means of a flexible PCB connection 24c, whereas in alternative variants of the present invention, a flexible PCB bent into a shape with two corresponding parts 24a and 24b may also be used instead.

Furthermore, the PCB 24 and the electronic components 22 in their entirety are encapsulated within an encapsulation material 26 which fills the entire compartment 14b in at least part of the longitudinal extension L of the handgrip 10, in which the PCB 24 is present. By employing an encapsulation material for the encapsulation, which is resilient to sterilization processes to which the handgrip is expected to be subjected during its regular usage cycles, it can be ensured that the electronics module is unaffected by such treatments and that its operation will not negatively be affected by repeated sterilization processes to which the handgrip is subjected.

## Claims

1. Electronics module (20), in particular sensor data logger, for integration into a handgrip (10) of a medical instrument, comprising:
- a two-part printed circuit board (24), PCB, wherein:
∘ a first part (24a) of the PCB (24) is at least as wide as a second part (24b) of the PCB (24),
∘ the two parts (24a, 24b) are arranged in such a manner that the second part (24b) is positioned in a plane different from the plane of the first part (24a),
∘ in a top view onto the first part (24a), the second part (24b) at least concerning a width direction (W) is contained within the width of the first part (24a), and
∘ the two parts (14a, 24b) are interconnected,
- electronic components (22) provided to the PCB (24); and
- an encapsulation material (26) encapsulating the PCB (24), wherein the encapsulation material (26) is resilient to sterilization processes.

2. Electronics module (20) according to claim 1,
further comprising at least one energy storage unit, in particular a battery, for example a primary coin cell, for powering the electronic components (22).

3. Electronics module (20) according to claim 2,
wherein the at least one energy storage unit is also encapsulated by the encapsulation material (26).

4. Electronics module (20) according to any of the preceding claims,
wherein the encapsulation material (26) is a silicone compound or a duromer plastic compound.

5. Electronics module (20) according to any of the preceding claims,
wherein the encapsulation material (26) is applied in a low-temperature process.

6. Electronics module (20) according to any of the preceding claims, wherein the electronic components (22) comprise an antenna system and a radio function for wireless communication with an external communication unit.

7. Electronics module (20) according to any of the preceding claims, wherein the electronic components (22) are adapted for a regular collection of sensor data by means of a sensor unit, in particular concerning temperature, pressure, humidity, acceleration, bending forces, mechanical forces and/or the presence of chemical substances.

8. Electronics module (20) according to any of the preceding claims, wherein the plane of the first part (24a) of the PCB (24) is parallel to the plane of the second part (24b) of the PCB (24).

9. Electronics module (20) according to any of the preceding claims, wherein the first part (24a) and the second part (24b) of the PCB (24) are interconnected at a longitudinal end region thereof, in particular by means of a flexible PCB connection (24c).

10. Handgrip (10) of a medical instrument, comprising:
- an outer shell part (12) delimiting an inner space (14) of the handgrip (10); and
- an electronics module (20) according to any of the preceding claims positioned in the inner space (14) of the handgrip (12).

11. Handgrip (10) according to the preceding claim,
further comprising a partitioning wall (16) inside the inner space (14) of the handgrip (10), which together with the outer shell part (12) defines a compartment (14b) of the inner space (14), in which the electronics module (20) is housed.

12. Handgrip (10) according to the preceding claim,
wherein the encapsulation material (26) substantially fills the entire compartment (14b) for at least a longitudinal section of the inner space (14) of the handgrip (10).

13. Handgrip (10) according to any of claims 10 to 12,
wherein the partitioning wall (16) is positioned inside the inner space (14) of the handgrip (10) in such a manner that in a cross-section view, the compartment (14b) accounts for less than half of the cross-section area of the inner space (14).

14. Handgrip (10) according to any of claims 10 to 13,
wherein the outer shell part (12) has a substantially circular cross-section.

15. Medical instrument, in particular video laparoscope, comprising a handgrip (10) according to any of claims 10 to 14.
